# EUROPEAN PATENT APPLICATION

(11) **EP 3 827 832 A1**
(43) Date of publication of application: **02.06.2021**
(21) Application number: 19858125.8
(22) Date of filing: 28.08.2019
(51) Int. Cl.: A61K 31/52, A61P 35/00

(54) **APPLICATION OF ALLOPURINOL IN PREPARATION OF DRUGS FOR TREATING PAICS GENE HIGHLY-EXPRESSED CANCERS**

(30) Priority: 03.09.2018 CN 201811018086
(71) Applicant: Geneheal Biotechnology Co., Ltd., Guangzhou, Guangdong 510000 (CN)
(72) Inventor: ZHU, Wei, Guangzhou, Guangdong 510000 (CN); PAN, Wuguang, Guangzhou, Guangdong 510000 (CN)
(74) Representative: Gassner, Birgitta
(86) International application number: PCT/CN2019/103050
(87) International publication number: WO 2020/048363

(57) **Abstract**

Disclosed is an application of allopurinol in the preparation of a drug for treating a cancer with high expression of a PAICS gene. Through screening by cell biology combined with animal experiments, and by bioinformatics analysis of gene expression of about 10,000 sample data from the TCGA database, It can be found and confirmed that allopurinol has significant anti-cancer and therapeutic effects on the cancer with abnormally high expression of the PAICS gene, including but not limited to, combined chemotherapy for cancer resistance, inhibition of cancer recurrence, improvement of prognosis survival rate, and the like.

## Description

### TECHNICAL FIELD

The present disclosure relates to a novel application of allopurinol, and in particular to an application of allopurinol in the preparation of a drug for treating a cancer with high expression of a PAICS gene.

### BACKGROUND

Tumors, especially malignant tumors, seriously threaten people's health. There are many pathogeneses of tumors. The different pathogeneses of different tumors lead to different responses of the tumors to different compounds. Chemotherapeutic drugs have certain therapeutic effects on most tumors mainly due to their broad-spectrum lethality to cells. However, compounds that do not have universal lethality to cells are often only effective to specific tumors, but not effective to other tumors, and even have the risk of promoting tumor progression. Therefore, the use of chemotherapeutic drugs is strictly limited.

*Allopurinol*, also known as zyloprim, is a drug that can inhibit a xanthine oxidase, so that hypoxanthine and xanthine cannot be converted into uric acid. That is, the synthesis of uric acid is reduced, thereby reducing the concentration of uric acid in blood, reducing the deposition of urate in bones, joints and kidneys, and being capable of inhibiting the synthesis of uric acid. Clinically, Allopurinol is generally used for:
1. primary and secondary hyperuricemia, especially for hyperuricemia patients with excessive production of uric acid, also for hyperuricemia with renal insufficiency;
2. treatment of gout, and is suitable for people with recurrent or chronic gout; when used for patients with gouty nephropathy, it can relieve the symptoms of gouty nephropathy, and can reduce the formation of urate calculi in kidney;
3. tophus; and
4. urate kidney calculi and/or urate nephropathy.

QIN Zhen, CHEN Chao. Advances in allopurinol research [J]. Chinese Pharmacological Bulletin, 2003, 19(11): 1220-1222 further disclosed that, with the continuous expansion of oxygen free radical theory, it was found that allopurinol could reduce the generation of oxygen free radicals in ischemia and reperfusion injury and thus achieves an antioxidant effect, so many researchers used allopurinol for treating the ischemia and reperfusion injuries at sites such as cardiovascular, brain, lung, stomach and intestine, and liver; furthermore, allopurinol also has the function of vascular dilation and provides a new drug for treating chronic heart failure; the effect of allopurinol on reducing uric acid synthesis can also be used for treating non-bacterial prostatitis and tumor lysis syndrome.

CN102746306A disclosed that allopurinol derivatives could effectively inhibit tumor growth and had good anti-tumor effects, and their activities were equivalent to that of 17-AAG which was undergoing the phase 3 clinical trial. In addition, allopurinol derivatives could also effectively inhibit the activity of xanthine oxidase, and could be used for the treatment of gout. The specific experimental data showed that allopurinol had no anti-tumor activity against Bel-7402 and SMMC-7221.

The full name of PAICS gene is phosphoribosylaminoimidazole carboxylase and phosphoribosylaminoimidazolesuccinocarboxamide synthase (https://www.ncbi.nlm.nih.gov/gene/10606), and its transcription and translation product is aminoimidazole succinylocarboxamide ribonucleotide synthetase. The PAICS gene participates in the 6th and 7th steps of purine biosynthesis.

### SUMMARY

The purpose of the present disclosure is to provide an application of allopurinol in the preparation of a drug for treating a cancer with high expression of a PAICS gene.

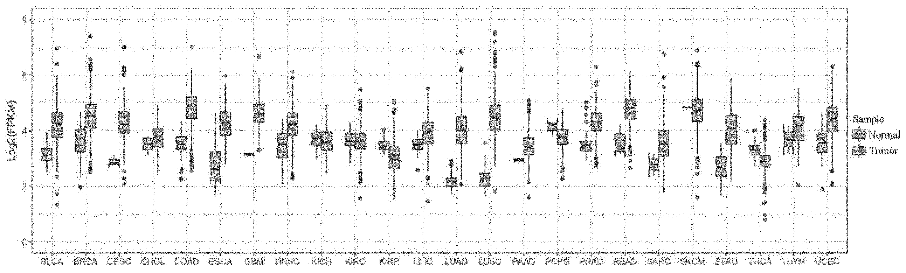

During the experiment, the inventor has found that allopurinol has significant anti-cancer and therapeutic effects on partial of tumor cells, but lacks significant anti-cancer and therapeutic effects on partial of the tumor cells. This property of allopurinol is different from those of most chemotherapeutic drugs. To further determine the reasons, by bioinformatics analysis of gene expression of about 10,000 sample data from the TCGA database (The Cancer Genome Atlas) in combination with further experiments, the inventor has found and confirmed that allopurinol has significant anti-cancer and therapeutic effects on the cancer with abnormally high expression of the PAICS gene, including but not limited to, combined chemotherapy for cancer resistance, inhibition of cancer recurrence, improvement of prognosis survival rate, and the like, as shown in FIG. 1, Table 1 and Table 2. Allopurinol can be used for treating cancers with abnormally high expression of the PAICS gene, such as lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma, but has no significant therapeutic effect on cancers with non-abnormally high expression of the PAICS gene such as renal cancer, skin cutaneous melanoma, pheochromocytoma and paraganglioma, which proves that allopurinol can be developed into a novel targeted anti-cancer drug against the cancer with abnormally high expression of the cancer gene PAICS.

In a first aspect of the present disclosure, provided is an application of allopurinol in the preparation of a drug for treating or preventing a cancer related to high expression of a PAICS gene.

In some embodiments of application, the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

In some embodiments of application, the drug for treating the cancer further comprises at least one existing anti-tumor drug.

In some embodiments of application, the treatment includes:
prevention of incidence of the cancer, especially incidence of the cancer in a population with abnormally high expression of the PAICS gene;
combined chemotherapy; and
prevention of recurrence of the cancer.

In a second aspect of the present disclosure, provided is a composition for treating the cancer related to high expression of the PAICS gene, where the active ingredients of the composition comprise at least one existing anti-tumor drug and allopurinol.

In some embodiments of the composition, the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

In a third aspect of the present disclosure, provided is a method for treating or preventing the cancer related to high expression of the PAICS gene, comprising administering a therapeutic or preventive amount of allopurinol to a patient.

In some embodiments of the method, the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

In a fourth aspect of the present disclosure, provided is allopurinol for use in the drug for preventing or treating the cancer related to high expression of the PAICS gene.

In some embodiments, the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

In some embodiments, the drug for treating the cancer further comprises at least one existing anti-tumor drug.

### BRIEF DESCRIPTION OF THE DRAWINGS

FIG. 1 shows the expression of the PAICS gene in different tumors.

### DETAILED DESCRIPTION

Through screening by cell biology combined with animal experiments, and by bioinformatics analysis of gene expression of about 10,000 sample data from the TCGA database (The Cancer Genome Atlas), It can be found that allopurinol has significant anti-cancer and therapeutic effects on the cancer with abnormally high expression of the PAICS gene, including but not limited to, combined chemotherapy for cancer resistance, inhibition of cancer recurrence, promotion of prognosis survival rate, and the like, as shown in FIG. 1, Table 1 and Table 2. Allopurinol can be used for treating cancers with abnormally high expression of the PAICS gene, such as lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma, but has no significant therapeutic effect on cancers with non-abnormally high expression of the PAICS gene such as renal cancer, skin cutaneous melanoma, pheochromocytoma and paraganglioma, which proves that allopurinol can be developed into a new targeted anti-cancer drug against the cancer with abnormally high expression of the cancer gene PAICS.

**Table 1. Anti-cancer effect of allopurinol and targeted association of high expression of the cancer gene PAICS**

| **Abbreviations of Tumors** | **English Name** | **Chinese Name** | **Anti-cancer effect of allopurinol** | **Expression of cancer gene PAICS** |
|---|---|---|---|---|
| BLCA | Bladder Urothelial Carcinoma | 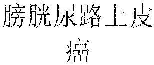 | Significant | Abnormally high expression |
| BRCA | Breast invasive carcinoma | 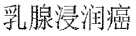 | Significant | Abnormally high expression |
| CESC | Cervical squamous cell carcinoma and endocervical adenocarcinoma | 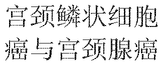 | Significant | Abnormally high expression |
| CHOL | Cholangiocarcinoma | 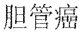 | Significant | Abnormally high expression |
| COAD | Colon adenocarcinoma | 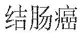 | Significant | Abnormally high expression |
| ESCA | Esophageal carcinoma | 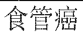 | Significant | Abnormally high expression |
| GBM | Glioblastoma multiforme | 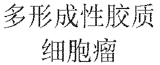 | Significant | Abnormally high expression |
| HNSC | Head and Neck squamous cell carcinoma | 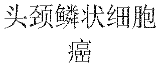 | Significant | Abnormally high expression |
| LIHC | Liver hepatocellular carcinoma | 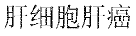 | Significant | Abnormally high expression |
| LUAD | Lung adenocarcinoma | 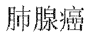 | Significant | Abnormally high expression |
| LUSC | Lung squamous cell carcinoma | 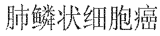 | Significant | Abnormally high expression |
| PAAD | Pancreatic adenocarcinoma | 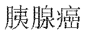 | Significant | Abnormally high expression |
| PRAD | Prostate adenocarcinoma | 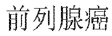 | Significant | Abnormally high expression |
| READ | Rectum adenocarcinoma | 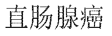 | Significant | Abnormally high expression |
| STAD | Stomach adenocarcinoma | 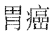 | Significant | Abnormally high expression |
| UCEC | Uterine Corpus Endometrial Carcinoma | 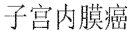 | Significant | Abnormally high expression |
| SKCM | Skin Cutaneous Melanoma | 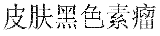 | Not significant | Non-abnormally high expression |
| KIRP | Kidney renal papillary cell carcinoma | 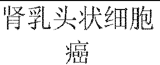 | Not significant | Non-abnormally high expression |
| PCPG | Pheochromocytoma and Paraganglioma | 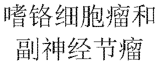 | Not significant | Non-abnormally high expression |

**Table 2. Table of results of bioinformatics analysis on gene expression of about 10,000 sample data from TCGA database**

| **Sample** | **Tumor** | **N** | **Fpkm** | **sd** | **se** | **ci** | **p** |
|---|---|---|---|---|---|---|---|
| Normal | BLCA | 19 | 9.327434674 | 2.672895246 | 0.613204224 | 1.28829427 | 2.20E-16 |
| Tumor | BLCA | 414 | 20.9776397 | 11.54552186 | 0.567431443 | 1.115413926 | 2.20E-16 |
| Normal | BRCA | 113 | 13.36097091 | 5.029916601 | 0.473174751 | 0.937535116 | 2.20E-16 |
| Tumor | BRCA | 1109 | 25.55254641 | 13.16364714 | 0.395285114 | 0.775591817 | 2.20E-16 |
| Normal | CESC | 3 | 7.588331772 | 0.900383976 | 0.519836931 | 2.23667779 | 1.07E-10 |
| Tumor | CESC | 306 | 21.66568741 | 11.84611489 | 0.677197314 | 1.332570153 | 1.07E-10 |
| Normal | CHOL | 9 | 11.41942575 | 1.945380049 | 0.648460016 | 1.49535148 | 0.02 |
| Tumor | CHOL | 36 | 14.16192433 | 5.546450019 | 0.924408337 | 1.876648693 | 0.02 |
| Normal | COAD | 41 | 11.54490736 | 3.29494978 | 0.514584702 | 1.040014478 | 2.20E-16 |
| Tumor | COAD | 480 | 30.71691167 | 12.58989725 | 0.57464756 | 1.129141574 | 2.20E-16 |
| Normal | ESCA | 11 | 8.452161586 | 6.443549375 | 1.942803236 | 4.328835372 | 2.89E-05 |
| Tumor | ESCA | 162 | 21.36681525 | 9.944839205 | 0.78134036 | 1.542997265 | 2.89E-05 |
| Normal | GBM | 5 | 8.873737015 | 0.246760588 | 0.11035469 | 0.306393739 | 5.27E-13 |
| Tumor | GBM | 169 | 28.87726013 | 33.19461096 | 2.553431612 | 5.040946876 | 5.27E-13 |
| Normal | HNSC | 44 | 11.48721302 | 4.342846942 | 0.65470881 | 1.320346151 | 2.20E-16 |
| Tumor | HNSC | 502 | 20.55320498 | 9.181093488 | 0.409772256 | 0.805083782 | 2.20E-16 |
| Normal | KICH | 24 | 13.35539875 | 2.680257976 | 0.547105368 | 1.131773684 | 9.90E-01 |
| Tumor | KICH | 65 | 13.36365723 | 5.104832408 | 0.633176533 | 1.264915536 | 9.90E-01 |
| Normal | KIRC | 72 | 12.78990784 | 2.734433004 | 0.32225602 | 0.642560254 | 7.60E-01 |
| Tumor | KIRC | 539 | 12.90381121 | 4.312682053 | 0.185760366 | 0.364904539 | 7.60E-01 |
| Normal | KIRP | 32 | 11.39360815 | 2.033659796 | 0.359503658 | 0.733212545 | 4.57E-06 |
| Tumor | KIRP | 289 | 9.153472515 | 4.701729025 | 0.276572296 | 0.54435932 | 4.57E-06 |
| Normal | LIHC | 50 | 11.60426532 | 2.149671354 | 0.304009438 | 0.610929839 | 2.20E-16 |
| Tumor | LIHC | 374 | 16.32540545 | 6.57659634 | 0.340067701 | 0.668690184 | 2.20E-16 |
| Normal | LUAD | 59 | 4.648806366 | 0.931580893 | 0.121281502 | 0.242771303 | 2.20E-16 |
| Tumor | LUAD | 535 | 18.57103446 | 10.57830203 | 0.457339878 | 0.898405936 | 2.20E-16 |
| Normal | LUSC | 49 | 5.058222705 | 1.488006106 | 0.212572301 | 0.427405257 | 2.20E-16 |
| Tumor | LUSC | 502 | 26.32049917 | 18.26619426 | 0.815260148 | 1.601750028 | 2.20E-16 |
| Normal | PAAD | 4 | 7.674271212 | 0.453850202 | 0.226925101 | 0.72217695 | 5.93E-11 |
| Tumor | PAAD | 178 | 11.696534 | 4.765053276 | 0.357155843 | 0.704831771 | 5.93E-11 |
| Normal | PCPG | 3 | 17.55543011 | 3.32020778 | 1.916922856 | 8.247853358 | 2.10E-01 |
| Tumor | PCPG | 183 | 14.10502517 | 4.376249847 | 0.323501697 | 0.638296048 | 2.10E-01 |
| Normal | PRAD | 52 | 12.33026969 | 4.673387071 | 0.648082181 | 1.301079269 | 2.20E-16 |
| Tumor | PRAD | 499 | 20.70739104 | 7.571625043 | 0.338952488 | 0.665953167 | 2.20E-16 |
| Normal | READ | 10 | 12.18742651 | 3.408283301 | 1.077793814 | 2.438138997 | 6.23E-12 |
| Tumor | READ | 167 | 29. 12261624 | 11.52792876 | 0.89205791 | 1.76124146 | 6.23E-12 |
| Normal | SARC | 2 | 7.141924857 | 2.700927032 | 1.90984382 | 24.26686658 | 1.60E-01 |
| Tumor | SARC | 263 | 13.52461268 | 9.430258738 | 0.581494665 | 1.144997719 | 1.60E-01 |
| Normal | SKCM | 1 | 28.60249926 | NA | NA | NA | NA |
| Tumor | SKCM | 471 | 28.64451085 | 13.30001465 | 0.612832326 | 1.204230335 | NA |
| Normal | STAD | 32 | 6.822764552 | 2.175404985 | 0.384560904 | 0.784317135 | 2.20E-16 |
| Tumor | STAD | 375 | 18.52699045 | 9.175342713 | 0.47381266 | 0.931670714 | 2.20E-16 |
| Normal | THCA | 58 | 9.995249023 | 1.871022752 | 0.245677323 | 0.491960354 | 1.07E-11 |
| Tumor | THCA | 510 | 7.87544214 | 2.297022908 | 0.101713884 | 0.199830712 | 1.07E-11 |
| Normal | THYM | 2 | 13.70575857 | 6.492251824 | 4.59071529 | 58.33056836 | 4.60E-01 |
| Tumor | THYM | 119 | 18.94238671 | 7.884432014 | 0.722764698 | 1.431270874 | 4.60E-01 |
| Normal | UCEC | 35 | 12.93212184 | 5.221415655 | 0.882580331 | 1.793619032 | 5.01E-14 |
| Tumor | UCEC | 552 | 22.93621572 | 10.61056013 | 0.45161543 | 0.887098565 | 5.01E-14 |

## Claims

1. An application of allopurinol in the preparation of a drug for treating or preventing a cancer related to high expression of a PAICS gene.

2. The application of claim 1, wherein the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

3. The application of claim 1 or 2, wherein the drug for treating the cancer further comprises at least one existing anti-tumor drug.

4. The application of any one of claims 1-3, wherein the treatment comprises:
prevention of incidence of the cancer, especially incidence of the cancer in a population with abnormally high expression of the PAICS gene;
combined chemotherapy; and
prevention of recurrence of the cancer.

5. A composition for treating the cancer related to high expression of the PAICS gene, wherein the active ingredients of the composition comprise at least one existing anti-tumor drug and allopurinol.

6. The composition of claim 5, wherein the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

7. A method for treating or preventing the cancer related to high expression of the PAICS gene, comprising administering a therapeutic or preventive amount of allopurinol to a patient.

8. The method of claim 7, wherein the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

9. Allopurinol for use in the drug for preventing or treating the cancer related to high expression of the PAICS gene.

10. The allopurinol of claim 9, wherein the cancer is selected from lung cancer, breast cancer, colon adenocarcinoma, rectal cancer, prostate adenocarcinoma, bladder cancer, cervical cancer, liver cancer, cholangiocarcinoma, esophageal cancer, glioblastoma multiforme, head and neck squamous cell carcinoma, pancreatic adenocarcinoma, stomach adenocarcinoma and uterine corpus endometrial carcinoma with high expression of the PAICS gene.

11. The allopurinol of claim 9 or 10, wherein the drug for treating the cancer further comprises at least one existing anti-tumor drug.
